# EUROPEAN PATENT APPLICATION

(11) **EP 3 892 741 A1**
(43) Date of publication of application: **13.10.2021**
(21) Application number: 20168593.0
(22) Date of filing: 07.04.2020
(51) Int. Cl.: C12Q 1/70, C12Q 1/6844

(54) **METHOD FOR A RAPID DETECTION OF A SARS-COV-2 INFECTION**

(71) Applicant: Eberhard Karls Universität Tübingen Medizinische Fakultät, 72074 Tübingen (DE)
(72) Inventor: BEZDAN, Daniela, 70184 Stuttgart (DE); IFTNER, Angelika, 72145 Hirrlingen (DE); POHLE, Diana, 72072 Tübingen (DE); IFTNER, Thomas, 72145 Hirrlingen (DE)
(74) Representative: Witte, Weller & Partner Patentanwälte mbB

(57) **Abstract**

The present invention relates to a method for a rapid detection of an infection or non-infection of a living being with SARS-CoV-2, a new starting material for said detection via loop-mediated isothermal amplification (LAMP), and to a kit for performing said method.

## Description

The present invention relates to a method for a rapid detection of an infection or non-infection of a living being with SARS-CoV-2, a new starting material for said detection via loop-mediated isothermal amplification (LAMP), and to a kit for performing said method.

### FIELD OF THE INVENTION

The present invention relates to the field of molecular biology, more particular to the field of molecular diagnostics.

### BACKGROUND OF THE INVENTION

Severe acute respiratory syndrome coronavirus 2 (SARS-CoV-2), colloquially known as the coronavirus and previously known by the provisional name 2019 novel coronavirus (2019-nCoV), is a positive-sense single-stranded RNA virus. It causes coronavirus disease 2019 (COVID-19), a respiratory illness. SARS-CoV-2 is contagious in humans, and the World Health Organization (WHO) has designated the ongoing pandemic of COVID-19 a Public Health Emergency of International Concern. The strain was first discovered in Wuhan, China, so it is sometimes referred to as the "Wuhan virus" or "Wuhan coronavirus". Because the WHO discourages the use of names based upon locations and to avoid confusion with the disease SARS, it sometimes refers to SARS-CoV-2 as "the COVID-19 virus" in public health communications. The general public frequently calls both SARS-CoV-2 and the disease it causes "coronavirus", but scientists typically use more precise terminology.

So far there is no appropriate therapy of COVID-19. Therefore, it is globally relied on early diagnostics and isolation of infected people. This approach attempts to slow down the spread of SARS-CoV-2.

Currently the diagnosis of an infection with SARS-CoV-2 is typically made by the use of RT-PCR allowing the direct detection of the virus, which provides reliable results, however, requires special laboratory equipment. Further, the duration from the sample collection until the obtainment of a result is quite lengthy. Such shortcomings makes RT-PCT inappropriate for using it as a point-of-use test, performed e.g. in the field such as in drive-in test stations.

Zhang et al. (2020), Rapid Molecular Detection of SARS-CoV-2 (COVID-19) Virus RNA Using Colorimetric LAMP, medRxiv preprint 2020.02.26.20028373, disclose the detection of SARS-CoV-2 virus RNA from purified RNA by loop-mediated isothermal amplification (LAMP) using a visual, colorimetric detection. By this method the duration of the test until the obtainment of a result can be significantly shortened. LAMP is a single-tube isothermal technique for the amplification of nucleic acids. In contrast to the polymerase chain reaction (PCR) technology, in which the reaction is carried out with a series of alternating temperature steps or cycles, isothermal amplification is carried out at a constant temperature, and does not require a thermal cycler. Further, in contrast to RT-PCT LAMP only requires heating and visual inspection. However, the method described by the authors requires the isolation and purification of RNA from the swab sample prior to subjecting it to the LAMP analysis. This requires laboratory equipment and, therefore, disqualifies the known method for a point-of-use test.

Against this background, it is an object underlying the invention to provide a method for a rapid detection of an infection or non-infection of a living being with SARS-CoV-2, where the disadvantages of the methods known in the art are avoided or at least reduced. Such a method should be suitable as a point-of-use test by obviating sophisticated laboratory equipment.

This object and other objects are met by the provision of a method for a rapid detection of an infection or non-infection of a living being with SARS-CoV-2, comprising the following steps:
1. Providing a swab sample from the living being suspected of being infected with SARS-CoV-2,
2. Subjecting the swab sample to loop-mediated isothermal amplification (LAMP),
3. Diagnosing the living being as being infected or non-infected on the basis of the color signal output of the LAMP,
characterized in that said swab sample is subjected to LAMP without performing a prior RNA extraction step.

The inventors have realized that the swab sample obtained from the living being can be directly subjected to loop-mediated isothermal amplification (LAMP) for subsequent analysis for the presence or absence of SARS-CoV-2-specific genetic material. Other than it is described by Zhang et al. (*loc. cit*.) the isolation of RNA from the swab sample is not necessary. In contrast, by the method according to the invention the crude swab sample can be directly used in the LAMP-based analysis.

The invention, therefore, qualifies for a field use without requiring complex equipment of a diagnostic laboratory.

According to the invention a "swab sample" is to be understood as a wiped-off biological material from the living being, potentially comprising SARS-CoV-2 specific material, such as nucleic acid, in particular RNA. In an embodiment of the invention the swab sample is a swab from the throat of the living being.

"Diagnosing" according to the invention refers to the determination whether or not the swab sample contains SARS-CoV-2 specific material. The diagnosis allows the conclusion whether the living being is infected by SARD-CoV-2 or not.

According to the invention "without performing a prior RNA extraction step" means that the crude swab sample can be used in the LAMP-based analysis and the RNA is not isolated before.

According the invention the "loop-mediated isothermal amplification" (LAMP) can be performed according to standard protocols known by the skilled person, e.g. in compliance with the manufacturer's instructions, preferably as basically described in Zhang et al. (*loc. cit*.)*,* however without a prior isolation of RNA from the swab sample but by directly using the crude swab sample.

According to the invention a "living being" refers to any living being subject to infections by SARS-CoV-2, such as mammals, including humans, but also bats, cats, dogs etc.

The object underlying the invention is herewith completely solved.

The inventors were able to verify that in comparison with the time consuming RT-PCT the method according to the invention has a specificity of at least 95.2% and a sensitivity of at least 75%. Such values are perfectly sufficient for a rapid test performed "in the field", such as drive-in test stations.

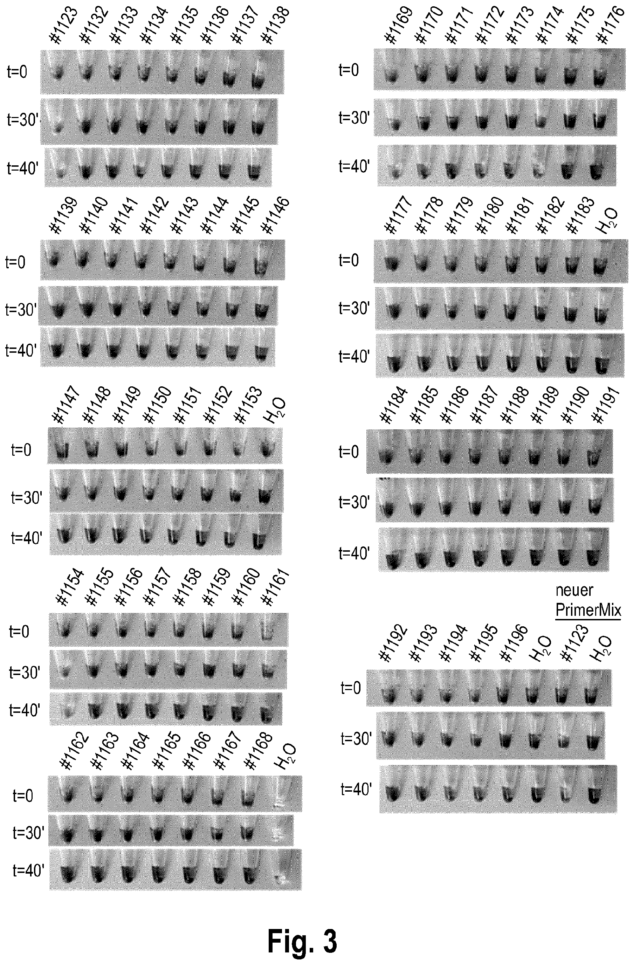

Even though Zhang et al. (*loc. cit*.) refers to performing a detection using "crude cell lysate in order to avoid an RNA purification step" it is clear for the skilled person when reviewing this document that the authors have in fact not used crude cell lysates, much less a crude swab samples. At first, the material-and-method part of this document is silent on the nature and obtainment of any "crude cell lysate". Second, in the legend of Fig. 3A it is referred to synthetic RNA that is added to the LAMP reaction but not to a crude cell lysate. Third, in the legend of Fig. 3A reference is made to Hela cells making the assertions of the authors even more questionable because Hela is an immortal cell line used in scientific research, which cannot even be infected by SARS-CoV-2.

Therefore, according to the believe of the inventors in the method according to the invention for the very first time a crude swab sample is used directly in an LAMP analysis without a prior RNA purification step.

In an embodiment of the invention said swab sample is provided on a dry carrier.

The inventors have realized that by using a dry carrier, such as a dry cotton tip or stick the method provides even more reliable results. This measure has, therefore, the advantage that the accuracy of the colorimetric signal obtained in the LAMP analysis is further increased.

In another embodiment the dry carrier is a dry cotton stick.

By this measure the method according to the invention is adapted to the needs of a rapid point-of-use test where cotton sticks are the tools of choice. It has turned out that the use of dry cotton sticks significantly improves the diagnostic certainty.

In a further embodiment of the invention the said swab sample is extracted from the carrier by a non-buffered liquid.

The inventors have surprisingly realized that buffered solutions interfere with the LAMP analysis and the colorimetric signal, respectively. The use of a non-buffered solution, successfully avoids this disadvantage. A "non-buffered" liquid is a liquid which is not qualified as a buffer solution, i.e. an aqueous solution consisting of a mixture of a weak acid and its conjugate base, or vice versa.

In another embodiment of the method according to the invention said swab sample is extracted from the carrier by an aqueous non-buffered liquid, preferably water, further preferably distilled water.

The inventors have realized that aqueous non-buffered liquid such as water or distilled water is the most preferred solvent for extracting the swab sample from the carrier, such as the cotton of the cotton stick. By using water the diagnostic certainty of the method according to the invention can be further increased.

In another embodiment of the invention the pH value of said swab sample is increased prior to the subjection of said sample to LAMP.

The inventors have realized increasing the pH value of the swab sample dispersed in the LAMP Master Mix (New England BioLabs; WarmStart Colorimetric LAMP 2X Master Mix; M1800) is also significantly improving the reliability and diagnostic certainty of the method according to the invention. Typically, the LAMP Master Mix does have a pH value of about 8.8 which, according to the findings of the inventors, is to be increased to improve the diagnostic outcome. In an embodiment of the invention the pH value of the swab sample is increased before the so-modified sample is subjected to LAMP and/or the LAMP Master Mix, respectively.

In another embodiment of the invention the pH value of said swab sample is increased to approx. 10 to 12, preferably approx. 10.5 to 11.5, further preferably of approx. 11, highly preferably of approx. 11.3.

The inventors have realized that the increase of the pH value of the swab sample dispersed in the LAMP Master Mix (see above) to the indicated ranges delivers optimum results.

In another embodiment of the invention the pH value of said swab sample is increased by the addition of sodium hydroxide (NaOH), preferably of 0.02 N NaOH.

The use of NaOH is advantageous for a field use as it is cheap and available without supply shortages. The indicated molar concentrations have turned out being suitable for a point-of-use test as amounts of liquids can be employed which are easy to handle.

In a further embodiment of the invention the living being is a human.

This measure has the advantage the method according to the invention is adapted to the needs of human medicine.

Another subject-matter of the invention relates to the use of a swab sample from a living being suspected of being infected with SARS-CoV-2 for a direct subjection to loop-mediated isothermal amplification (LAMP) without performing a prior RNA extraction step for a rapid detection of an infection or non-infection with SARS-CoV-2.

The features, characteristics, advantages and embodiments described for the method according to the invention apply to the use according to the invention correspondingly.

Another subject-matter of the invention relates to a kit for rapid detection of an infection or non-infection of a living being with SARS-CoV-2, comprising reagents and a manual for preforming the method according to the invention.

A "kit" is a combination of individual elements useful for carrying out the method of the invention, wherein the elements are optimized for use together in the methods. The kit may also contain additional reagents, chemicals, buffers, reaction vials etc. which may be useful for carrying out the method according to the invention, such as LAMP-specific reagents. Such a kit unifies all essential elements required to work the method according to the invention, thus minimizing the risk of errors. Therefore, such kits also allow semi-skilled laboratory staff to perform the method according to the invention.

The features, characteristics, advantages and embodiments described for the method according to the invention apply to the kit according to the invention correspondingly.

Therefore, in an embodiment of the invention the kit comprises a non-buffered liquid, preferably water, further preferably distilled water.

In another embodiment of the invention the kit comprises an alkaline solution, preferably sodium hydroxide (NaOH), preferably a solution of 0.02 N NaOH.

In still another embodiment the kit according to the invention comprises LAMP primer sets.

It is to be understood that the before-mentioned features and those to be mentioned in the following cannot only be used in the combination indicated in the respective case, but also in other combinations or in an isolated manner without departing from the scope of the invention.

### EMBODIMENTS

The invention is now further explained by means of embodiments resulting in additional features, characteristics and advantages of the invention. The embodiments are of pure illustrative nature and do not limit the scope or range of the invention. The features mentioned in the specific embodiments are features of the invention and may be seen as general features which are not applicable in the specific embodiment but also in an isolated manner in the context of any embodiment of the invention.

The invention is now further described and explained in further detail by referring to the following non-limiting examples and figures:
- Fig. 1: shows the outcome of the method according to the invention performed with one exemplary swab sample where the pH value was increased before the LAMP Master Mix was added;
- Fig. 2: shows the outcome of the method according to the invention performed with a larger number of swab samples;
- Fig. 3: shows the outcome of the method according to the invention performed with a still larger number of swab samples.

### 1. Colorimetric LAMP

The colorimetric LAMP was performed according to the manual of New England Biolabs. In brief:

### Materials

| Material | Concentration | Lot |
|---|---|---|
| NEB M1800L LAMP Mix | 2X | 10069263 |
| CoV-2 Gene N LAMP Primer Mix | 25X | Customized |

### Basic Protocol

1) Thaw LAMP Master Mix and LAMP Primer Mix at room temperature.
2) Mix thoroughly by vortexing or inversion, ensure that any precipitation in the M1800 mix is resuspended.
3) Assemble reactions using the following recipe:

| Material | Concentration | Volume (per 25 uL Reaction) |
|---|---|---|
| M1800 LAMP Mix | 2X | 12.5 µL |
| LAMP Primer Mix | 25X | 1 µL |
| Sample | - | As desired |
| Water | - | To 25 µL |

4) Verify reactions are pink; if yellow, repeat with lower sample volume or sample adjusted to pH ∼8.
5) Place reactions at 65 °C, incubate for 30 minutes. *NOTE:* For high copy number samples, time can be shortened to 15-20 minutes. For very low copy samples or maximum sensitivity, extend time to 40 minutes.
6) Inspect reaction tubes for yellow color. Allow reactions to cool from 65°C by placing at room temperature for improved color contrast, or place tubes on ice for 5 seconds before inspecting.
7) Discard completed reactions without opening reaction vessels.

### Primer Sequences (5'-3')

### Gene N

GeneN-F3 TGGCTACTACCGAAGAGCT (SEQ ID No. 1)
GeneN-B3 TGCAGCATTGTTAGCAGGAT (SEQ ID No. 2)
GeneN-FIP TCTGGCCCAGTTCCTAGGTAGTCCAGACGAATTCGTGGTGG (SEQ ID No. 3)
GeneN-BIP AGACGGCATCATATGGGTTGCACGGGTGCCAATGTGATCT (SEQ ID No. 4)
GeneN-LF GGACTGAGATCTTTCATTTTACCGT (SEQ ID No. 5)
GeneN-LB ACTGAGGGAGCCTTGAATACA (SEQ ID No. 6)

### 2. RT-PCR

RT-PCR as comparison method was performed via QIASymphona/AI-tona, as described by the instructions of the manufacturer.

### 3. Proof of principle

LAMP 30 min incubated at 65°C, dry swabs in 2ml PBS
- BS sample without boiling is very bad. Pos1/4 pos.
- PBS 5min 98 ° C significantly better -> 3/4 pos.
- Extracted RNA 4/4 pos.

LAMP 40 min incubated at 65°C
- PBS and PBS 98 ° C no difference
- RNA 7/10 RT PCR negative samples are weakly pos. In the LAMP
- Water was clearly negative even at 40 min
- CP 33 and 38 also become weakly pos. at 40 min

It turned out that PBS buffer is not appropriate for the colorimetric LAMP assay. The results are getting better after boiling but are still not satisfactory.

### 4. Swab dissolved in transport medium

- The inventors have taken swabs from the E-Swab diagnostics, of which the liquid has already been pipetted into storage tubes
- The remaining liquid was pipetted into Eppendorf tube and boiled at 98 °C for 5 minutes
- The swabs from the smear tubes were "squeezed out" in a 0.5 ml Eppendorf tubes. These Eppendorf tubes were also heated at 98 °C.
- When the LAMP assay reaction was added, the color changed immediately

It turned out that the E-swab transport medium is not suitable for LAMP assay.
- The swab 7259441256 from the above-mentioned tubes is soaked with 0.25 ml of water and shaken for 30 minutes.
- Liquid transferred to Eppendorf tubes and 5min 98 °C -> ice
- 10µl of this liquid to LAMP assay

Result after LAMP assay: yellow
-> Worked well. But too complicated

Transport medium is not suitable for LAMP-based method according to the invention. If diluted with water the outcome is better but still not suitable for a field application because of complication of the method.

### 5. Swab dissolved in various buffers

The inventors tested the following buffers
Buffer A: 10mM Tris pH 7.4, 0.5% NP40, 100mM NaCl, 1% RNAsin (Promega)
Buffer B: 10mM Tris pH 7.4, 0.5% Triton X100, 100mM NaCl, 1% RNAsin (Promega)
Buffer C: 10mM Tris pH 7.4, 0.5% Tween 20, 100mM NaCl, 1% RNAsin (Promega)

There is an additional reaction with Qiagen protease for each buffer.

None of the buffers work in the LAMP-based method according to the invention.

### 6. Swab dissolved in water, increase of pH value

Cotton swabs from the fairground samples:
- Dry swabs are transferred to 15 ml BlueCap
- Add 0.5 ml H2O, shake for 30 min
- H₂O "supernatant" is transferred to 2 ml screw Eppendorf tubes, swabs in BlueCap also at -80 °C

QIASymphony/Altona: 50 µl of the "supernatants" + 250 µl H₂O + 200 µl PBS
Altona RT-PCR made with all 79 samples
LAMP assay: 10 µl of the supernatants + 15 µl LAMP-MM
Direct color change for many samples BEFORE the reaction starts
Try to raise the pH again by adding NaOH

| Row | µl | xN NaOH | color reaction |
|---|---|---|---|
| A + K6 | 0,5 | 0,1 | bright pink |
| B + C | 1,0 | 0,01 | pink |
| D | 1,0 | 0,001 | orange |
| E-K | 1,0 | 0,01 | pink |

### Test whether increasing the pH value BEFORE adding the LAMP master mix enables a functional LAMP reaction

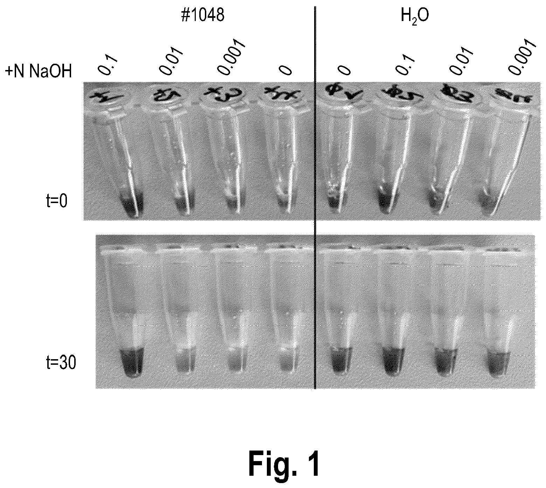

An original tube (PBS-shaken cotton swab (dry sample), fixed place # 1048 (CP = 15) is used as a sample
- 10 µl sample / H₂O + 1 µl 0.1 / 0.01 / 0.001 N NaOH or H₂O for 0 N NaOH (increase of pH)
- 5 min, 98 ° C, 4 °C ∞
- + 14 µl LAMP master mix consisting of:
   o 1 µl 25x primer mix
   o 12.5 µl 2xMM
   o 0.5 µl H2O
- LAMP reaction (65 ° C, 30 min; 4 ° C ∞)
- > by increasing the pH value BEFORE boiling, the LAMP reaction runs smoothly without problems. The addition of 0.01 - 0.001 N NaOH gives good results, resulting in a final pH of about 11 (see Fig. 1).

### LAMP test of the fairground samples from 03. April 2020 (part 1)

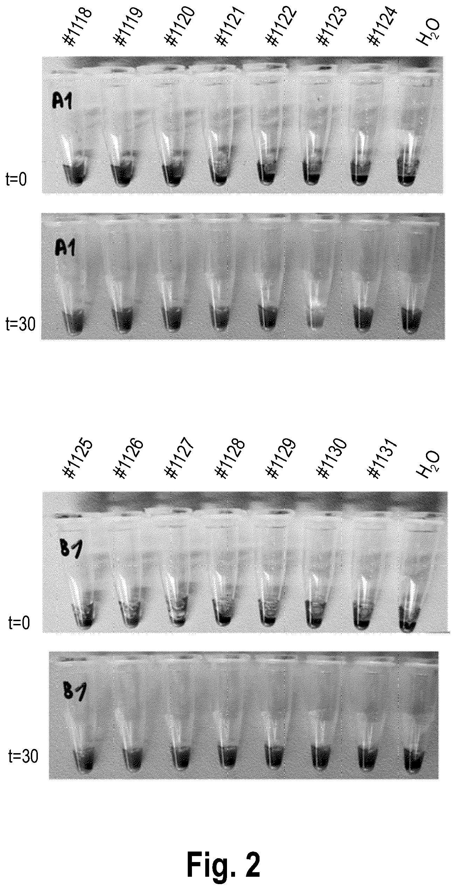

the original tubes (H₂O-shaken cotton swabs, fixed place #1118 to #1131 are used as samples
- 10 µl sample / H₂O + 1 µl 0.01 N NaOH (up to pH of approx. 11)
- 5 min, 98 ° C, 4 ° C ∞
- + 14 µl LAMP master mix consisting of:
   ∘ 1 µl 25x primer mix
   ∘ 12.5 µl 2xMM
   ∘ 0.5 µl H₂O
- LAMP reaction (65 ° C, 30 min; 4 ° C ∞)
- > by increasing the pH value BEFORE boiling, the LAMP reaction runs smoothly without problems. The addition of 0.01 N NaOH gives good results, resulting in a final pH of about 11 (see Fig. 2).

LAMP test of the fairground samples from 03. April 2020 (part 2)

the original tubes (H₂O-shaken cotton swabs, fixed place #1132 to #1196 are used as samples.

The method is performed as described for #1118 to #1131.
- > by increasing the pH value BEFORE boiling, the LAMP reaction runs smoothly without problems. The addition of 0.01 N NaOH gives good results, resulting in a final pH of about 11 (see Fig. 3).

### 7. Specificity and sensitivity of method according to the invention

The inventors have tested a large number of swab samples by the method according to the invention (dry cotton sticks, sample extracted by distilled water, pH increased to 11.3 by the addition of 0.02 N NaOH) and determined the diagnostic reliability by comparing the results with such obtained by RT-PCR.

| Ser.no. | RT PCR | E Gene | S Gene | | LAMP(N) | LAMP(N) | LAMP(N) | LAMP(N) | LAMP(ORF) | LAMP(ORF) | LAMP(ORF) |
|---|---|---|---|---|---|---|---|---|---|---|---|
| 1119 | questionable | neg | 37,64 | | neg | | | | | | |
| 1159 | questionable | 35,59 | neg | | neg | | | | | | |
| 1167 | questionable | 34,74 | neg | | neg | | | | | | |
| 1003 | neg | | | | neg | | | | | | |
| 1004 | neg | | | | neg | | | | | | |
| 1005 | neg | | | | neg | | | | | | |
| 1006 | neg | | | | neg | | | | | | |
| 1007 | neg | | | | neg | | | | | | |
| 1008 | neg | | | | neg | | | | | | |
| 1009 | neg | | | | neg | | | | | | |
| 1011 | neg | | | | neg | | | | | | |
| 1012 | neg | | | | neg | | | | | | |
| 1017 | neg | | | | neg | | | | | | |
| 1118 | neg | neg | neg | | neg | | | | | | |
| 1120 | neg | neg | neg | | neg | | | | | | |
| 1121 | neg | neg | neg | | neg | | | | | | |
| 1122 | neg | neg | neg | | neg | | | | | | |
| 1124 | neg | neg | neg | | neg | | | | | | |
| 1125 | neg | neg | neg | | neg | | | | | | |
| 1126 | neg | neg | neg | | neg | | | | | | |
| 1127 | neg | neg | neg | | neg | | | | | | |
| 1128 | neg | neg | neg | | neg | | | | | | |
| 1129 | neg | neg | neg | | neg | | | | | | |
| 1130 | neg | neg | neg | | neg | | | | | | |
| 1131 | neg | neg | neg | | neg | | | | | | |
| 1132 | neg | neg | neg | | neg | | | | | | |
| 1133 | neg | neg | neg | | neg | | | | | | |
| 1134 | neg | neg | neg | | +/- | | | | neg | pos | +/- |
| 1135 | neg | neg | neg | | neg | | | | | | |
| 1136 | neg | neg | neg | | neg | | | | | | |
| 1137 | neg | neg | neg | | neg | | | | | | |
| 1138 | neg | neg | neg | | neg | | | | | | |
| 1139 | neg | neg | neg | | neg | | | | | | |
| 1140 | neg | neg | neg | | neg | | | | | | |
| 1141 | neg | neg | neg | | neg | | | | | | |
| 1142 | neg | neg | neg | | neg | | | | | | |
| 1143 | neg | neg | neg | | neg | neg | neg | | | | |
| 1144 | neg | neg | neg | | neg | neg | neg | | | | |
| 1145 | neg | neg | neg | | neg | | | | | | |
| 1146 | neg | neg | neg | | neg | | | | | | |
| 1147 | neg | neg | neg | | neg | | | | | | |
| 1148 | neg | neg | neg | | neg | | | | | | |
| 1149 | neg | neg | neg | | neg | | | | | | |
| 1151 | neg | neg | neg | | neg | neg | neg | | | | |
| 1152 | neg | neg | neg | | neg | | | | | | |
| 1153 | neg | neg | neg | | neg | neg | neg | | | | |
| 1155 | neg | neg | neg | | neg | | | | | | |
| 1156 | neg | neg | neg | | neg | | | | | | |
| 1157 | neg | neg | neg | | neg | | | | | | |
| 1158 | neg | neg | neg | | neg | neg | neg | | | | |
| 1160 | neg | neg | neg | | neg | | | | | | |
| 1161 | neg | neg | neg | | neg | neg | neg | | | | |
| 1162 | neg | neg | neg | | neg | | | | | | |
| 1163 | neg | neg | neg | | neg | | | | | | |
| 1164 | neg | neg | neg | | neg | | | | | | |
| 1165 | neg | neg | neg | | neg | | | | | | |
| 1166 | neg | neg | neg | | neg | | | | | | |
| 1168 | neg | neg | neg | | neg | | | | | | |
| 1169 | neg | neg | neg | | +/- | neg | neg | | | | |
| 1171 | neg | neg | neg | | neg | | | | | | |
| 1172 | neg | neg | neg | | +/- | neg | neg | | | | |
| 1173 | neg | neg | neg | | +/- | neg | neg | | | | |
| 1175 | neg | 31,9 | neg | | neg | | | | | | |
| 1177 | neg | neg | neg | | neg | | | | | | |
| 1178 | neg | neg | neg | | neg | | | | | | |
| 1179 | neg | neg | neg | | neg | | | | | | |
| 1180 | neg | neg | neg | | neg | | | | | | |
| 1181 | neg | neg | neg | | neg | | | | | | |
| 1182 | neg | neg | neg | | neg | | | | | | |
| 1183 | neg | neg | neg | | neg | | | | | | |
| 1184 | neg | neg | neg | | neg | | | | | | |
| 1185 | neg | neg | neg | | neg | | | | | | |
| 1186 | neg | neg | neg | | neg | | | | | | |
| 1187 | neg | neg | neg | | neg | | | | | | |
| 1188 | neg | neg | neg | | neg | | | | | | |
| 1189 | neg | neg | neg | | neg | | | | | | |
| 1190 | neg | neg | neg | | neg | | | | | | |
| 1191 | neg | neg | neg | | neg | | | | | | |
| 1192 | neg | neg | neg | | neg | | | | | | |
| 1193 | neg | neg | neg | | neg | neg | neg | | | | |
| 1195 | neg | neg | neg | | neg | neg | neg | | | | |
| 1196 | neg | neg | neg | | +/- | neg | neg | | | | |
| 1048 | pos | 15,25 | | | pos | pos | pos | pos | | | |
| 1123 | pos | 21,29 | 27,56 | | pos | pos | pos | pos | pos | pos | pos |
| 1150 | pos | 29,03 | 30,27 | | neg | neg | neg | | | | |
| 1154 | pos | 20,29 | 21,96 | | pos | | | | pos | pos | pos |
| 1170 | pos | 22,86 | 27,53 | | +/- | | | | neg | neg | neg |
| 1174 | pos | 26,5 | 27,89 | | pos | | | | neg | +/- | neg |
| 1176 | pos | 26,4 | 28,6 | | neg | | | | neg | neg | neg |
| 1194 | pos | 28,42 | 28,85 | | +/- | +/- | neg | | neg | neg | neg |

It turned out that the method according to the invention has a specificity approx. 95.2% and a sensitivity in reference to the RT-PCR of 75%.

## Claims

1. Method for a rapid detection of an infection or non-infection of a living being with SARS-CoV-2, comprising the following steps:
1. Providing a swab sample from the living being suspected of being infected with SARS-CoV-2,
2. Subjecting the swap sample to loop-mediated isothermal amplification (LAMP),
3. Diagnosing the living being as being infected or non-infected on the basis of the color signal output of the LAMP,
**characterized in that** said swab sample is subjected to LAMP without performing a prior RNA extraction step.

2. The method of claim 1, **characterized in that** said swab sample is provided on a dry carrier.

3. The method of claim 2, **characterized in that** the dry carrier is a dry cotton stick.

4. The method of any of claim 2 or 3, **characterized in that** said swab sample is extracted from the carrier by a non-buffered liquid.

5. The method of any of claims 2 to 4, **characterized in that** said swab sample is extracted from the carrier by an aqueous non-buffered liquid, preferably water, further preferably distilled water.

6. The method of any of claims 1 to 5, **characterized in that** the pH value of said swab sample is increased prior to the subjection to LAMP.

7. The method of claim 6, **characterized in that** the pH value of said swab sample is increased to approx. 10 to 12, preferably approx. 10.5 to 11.5, further preferably of approx. 11, highly preferably of approx. 11.3.

8. The method of claim 6 or 7, **characterized in that** the pH value of said swab sample is increased by the addition of sodium hydroxide (NaOH), preferably of 0.02 N NaOH.

9. The method of any of the preceding claims, **characterized in that** the living being is a human.

10. Use of a swab sample from a living being suspected of being infected with SARS-CoV-2 for a direct subjection to loop-mediated isothermal amplification (LAMP) without performing a prior RNA extraction step for a rapid detection of an infection or non-infection with SARS-CoV-2.

11. Kit for rapid detection of an infection or non-infection of a living being with SARS-CoV-2, comprising reagents and a manual for preforming the method according to any of claims 1 to 9.

12. Kit of claim 11, **characterized in that** it comprises a non-buffered liquid, preferably water, further preferably distilled water.

13. Kit of claims 11 and 12, **characterized in that** it comprises an alkaline solution, preferably sodium hydroxide (NaOH), preferably a solution of 0.02 N NaOH.

14. Kit of claims 12 or 13, **characterized in that** it comprises LAMP primer sets.


SEQUENCE LISTING

<110>  Eberhard-Karls-Universtät, Universitätsklinikum

<120>  Method for a rapid detection of an SARS-CoV-2 infection

<130>  5402P606EP

<160>  6

<170>  PatentIn version 3.5

<210>  1
<211>  19
<212>  DNA
<213>  Artificial sequence

<220>
<223>  Primer GeneN-F3

<400>  1
tggctactac cgaagagct                                                    19


<210>  2
<211>  20
<212>  DNA
<213>  Artificial sequence

<220>
<223>  Primer GeneN-B3

<400>  2
tgcagcattg ttagcaggat                                                   20


<210>  3
<211>  41
<212>  DNA
<213>  Artificial sequence

<220>
<223>  Primer GeneN-FIP

<400>  3
tctggcccag ttcctaggta gtccagacga attcgtggtg g                           41


<210>  4
<211>  40
<212>  DNA
<213>  Artificial sequence

<220>
<223>  Primer GeneNBIP

<400>  4
agacggcatc atatgggttg cacgggtgcc aatgtgatct                             40


<210>  5
<211>  25
<212>  DNA

<213>  Artificial sequence

<220>
<223>  Primer GeneN-LF

<400>  5
ggactgagat ctttcatttt accgt                                              25


<210>  6
<211>  21
<212>  DNA
<213>  Artificial sequence

<220>
<223>  Primer GeneN-LB

<400>  6
actgagggag ccttgaatac a                                                  21